Europäisches Patentamt

European Patent Office

Office européen des brevets

(11) Numéro de publication: **0 406 137 A1**

# DEMANDE DE BREVET EUROPEEN

(21) Numéro de dépôt: 90420307.2

(22) Date de dépôt: 28.06.90

(51) Int. Cl.⁵: **G01N 15/10**, G06K 9/46, A01K 67/00, A01H 1/04

(30) Priorité: 30.06.89 FR 8909081

(43) Date de publication de la demande:
02.01.91 Bulletin 91/01

(84) Etats contractants désignés:
AT BE CH DE DK ES FR GB GR IT LI LU NL SE

(71) Demandeur: **UNION DES COOPERATIVES D'ELEVAGE ALPES ET RHONE BEL AIR**

**F-69340 Francheville(FR)**

(72) Inventeur: **Celerier, Bernard**
**Rue de Chapoly**
**F-69290 Saint-Genis-Les Ollieres(FR)**
Inventeur: **Brugal, Gérard**
**11 Avenue du Vercors**
**F-38420 Meylan(FR)**
Inventeur: **Monet, Jean-Dominique**
**8 Rue Camille Desmoulins**
**F-38400 Saint Martin d'Heres(FR)**

(74) Mandataire: **Monnier, Guy et al**
**Cabinet Monnier 150 Cours Lafayette B.P. 3058**
**F-69393 Lyon Cédex 03(FR)**

(54) Procédé de sélection rapide d'espèces, de variétés, de lignées ou d'individus animaux ou végétaux pour tout caractère génétique.

(57) Il consiste à appliquer l'analyse statistique d'image de noyaux cellulaires et du faciès chromatinien à l'estimation d'une information génétique. Cette information génétique est contenue parmi les paramètres de morphologie, de densitométrie, de texture de la chromatine et de couleur. Le procédé comporte également une étape d'analyse multiparamétrique permettant de déterminer les vecteurs les plus discriminants conduisant à l'établissement d'un indice de sélection. Ce procédé trouve des applications dans la sélection des espèces animales et des espèces végétales.

## LA PRÉSENTE INVENTION CONCERNE UN PROCÉDÉ DE SÉLECTION RAPIDE D'ESPÈCES, DE VARIÉTÉS, DE LIGNÉES OU D'INDIVIDUS, ANIMAUX OU VÉGÉTAUX, POUR TOUT CARACTÈRE GÉNÉTIQUE.

Dans ce qui va suivre, ce procédé sera plus particulièrement décrit dans ses applications dans la sélection de races bovines, en particulier dans le cas des reproducteurs utilisés en insémination artificielle, sans qu'il en résulte nullement une limitation à sa portée.

On sait toute l'importance qu'attache l'éleveur à réaliser une adaptation aussi satisfaisante que possible des bovins qu'il entretient aux buts pour lesquels il les exploite (production de lait, de viande ou de travail). Pendant longtemps la détection des meilleurs reproducteurs a reposé sur des méthodes de sélection empiriques à action fort lente, et le plus souvent imprécises.

L'acquisition de connaissances nouvelles dans le domaine de la génétique des populations, la mise au point et la pratique des techniques d'insémination ont constitué un progrés décisif pour l'amélioration de l'èlevage. A l'heure actuelle, 70% des femelles sont fécondées par insémination artificielle.

Les techniques de conservation des semences à très basse température permettent d'assurer un stockage très important, pouvant atteindre ou dépasser 200 000 doses de semence par reproducteur. Elles ont considérablement augmenté la capacité reproductrice des mâles, mais elles ont également rendu possible la mise à l'épreuve sur la descendance de ces géniteurs, le stockage de la semence sur une longue période, l'accouplement entre reproducteurs éloignés dans le temps et dans l'espace, ainsi qu'une diffusion massive des gènes des meilleurs reproducteurs. '

On conçoit facilement, compte-tenu de la quantité des doses stockées, tous les soins qu'il convient d'apporter à lé sélection des reproducteurs afin de ne diffuser massivement que celles des meilleurs.

Le choix des reproducteurs consiste actuellement à retenir ceux des candidats présentant le plus de chance de laisser une descendance supérieure, et ceci notamment pour les taureaux destinés à l'insémination artificielle.

Les méthodes appliquées pour la sélection des reproducteurs consistent à prendre en compte l'ensemble des informations disponibles sur ceux-ci ou leurs apparentés.

Cet ensemble d'opérations est regroupé sous les termes génériques suivants :
- informations ayant trait à l'ascendance : sélection sur ascendance
- informations ayant trait à l'individu lui-même : sélection individuelle
- informations ayant trait aux collatéraux : sélection collatérale
- informations ayant trait aux descendants : sélection sur la descendance.

La prise en compte de l'ensembmle des informations disponibles sur l'animal lui-même, ses ascendants, et/ou ses descendants, collectées soit en ferme soit dans le cadre de stations, permet d'établir, par l'application de modèles mathématiques adaptés, la qualité génétique de chaque reproducteur.

Les informations nécessaires pour estimer cette valeur génétique des candidats reproducteurs portent actuellement sur la collecte et le traitement des données découlant du contrôle de leurs performances connues au moment de l'estimation.

On calcule, pour chaque critère de sélection, c'est-à-dire pour un caractère ou une combinaison de caractères de sélection retenus, un index ou indice de sélection ; celui-ci constitue la meilleure estimation, à un moment donné, de la valeur génétique d'un reproducteur. Il s'agit, bien entendu, non pas d'une estimation dans l'absolu mais seulement d'une estimation relative de la valeur des candidats les uns par rapport aux autres, estimation obtenue en comparant, pour un caractère donné, la descendance du candidat à celle de ses contemporains.

L'estimation de la valeur génétique des candidats à la reproduction, à partir des informations collectées, constitue donc une opération fondamentale de la sélection. C'est ainsi par exemple que, dans la sélection des bovins de race laitière, les critères de sélection les plus couramment utilisés concernent la quantité de lait, la quantité moyenne de matière utile ou les taux de matière utile.

A titre indicatif, les programmes intégrés d'amélioration génétique mis en oeuvre en insémination artificielle reposent classiquement sur les étapes suivantes :
- Procréation de veaux mâles, par accouplements dirigés ou raisonnés entre reproducteurs de valeur génétique connue (1° tri sur ascendance)
- Contrôle de ces veaux mâles en station d'élevage ou de contrôle individuel (on note à ce stade une élimination d'environ 50% des sujets)
- Mise à l'épreuve de la descendance, qui consiste à inséminer un nombre limité de vaches afin d'obtenir le nombre de produits nécessaires au calcul de l'indice de sélection
- Récolte, conditionnement et stockage de la semence pendant la période de mise à l'épreuve et d'attente des résultats, le nombre de doses ainsi préparées et conservées étant variable selon les races et les unités de sélection

- Choix des meilleurs reproducteurs, sur leurs index de production
- Utilisation des reproducteurs, notamment pour la procréation de ceux de la génération suivante.

Tout ceci correspond bien évidemment à des programmes fort coûteux et surtout très longs que l'on peut résumer dans le tableau suivant, établi dans le cas d'un programme laitier :

TABLEAU 1

|  | DUREE | ANNEE |
|---|---|---|
| Choix des pères et mères à taureaux |  | 0 |
| Naissance des veaux | 9 mois | 9 mois |
| Elevage des veaux - Sélection individuelle | 19 mois | 2 ans 4 mois |
| Mise à l'épreuve des taureaux | 12 mois | 3 ans 4 mois |
| Naissance des filles | 12 mois | 4 ans 4 mois |
| Elevage des filles et fécondation | 20 mois | 6 ans |
| Vélage des filles | 12 mois | 7 ans |
| Contrôle des filles (production, conformation facilité de traite) calcul des index | 12 mois | 8 ans |
| Utilisation des taureaux | IMMEDIATE | 9 ANS |

Il faut donc compter en moyenne neuf années pour que le cycle soit complet. Le nombre de reproducteurs retenus à la connaisance des résultats est d'environ un sur cinq ; le prix de revient des reproducteurs retenus se situe, à l'heure actuelle, à 1.500.000 francs ; la sélection est d'une bonne fiabilité et l'intervalle de confiance varie entre 20 à 30%.

L'amélioration génétique constitue l'investissement fondamental de l'élevage. Sa place est de plus en plus importante dans la formation du revenu de l'éleveur et la mise à disposition des meilleurs géniteurs devrait pouvoir être assurée dans les délais les plus courts possibles, tout en satisfaisant aux exigences légitimes sur la qualité des géniteurs mis à la disposition des éleveurs.

La présente invention s'est donnée pour but de proposer un procédé simple et rapide permettant de déterminer dans un laps de temps relativement court le potentiel génétique des reproducteurs pour en réaliser plus rapidement la sélection et diminuer le coût de cette sélection.

Les inventeurs ont pu déterminer qu'il étant possible d'établir un lien valable et reproductible entre certains paramètres du noyau cellulaire (morphologie, densitométrie, texture de la chromatine, couleur...) et certains caractères génétiques d'espèces, de variétés, de lignées ou d'individus, animaux ou végétaux.

On sait que la chromatine constitue la structure supérieure du matériel génétique, structure dans laquelle l'ADN est associé à des protéines.

Cette chromatine est en fait un ensemble complexe d'ADN, de protéines basiques, dites histones, de protéines non-histones et d'ARN. On distingue souvent deux formes de chromatine dans le chromosome interphasique : l'une, extrêmement diffuse, de coloration faible, appelée euchromatine (ou chromatine décondensée) et l'autre, plus dense et de coloration plus intense, appelée hétérochromatine (ou chromatine condensée).

Certains systèmes d'analyse microscopique à balayage automatique utilisés en cytométrie et en histométrie permettent par ailleurs de réaliser une analyse fine de la structure de cette chromatine.

Cette analyse fine de la chromatine est largement utilisée à l'heure actuelle pour individualiser des populations ou des individus. Citons par exemple l'article de GIROUD F. et al. dans Cytométry 9:339-348-(1988)p. 339 - 348.

Or les inventeurs ont déterminé que ces méthodes d'analyse fine de la structure de la chromatine pouvaient également être appliquées à la sélection d'espèces, de variétés, de lignées ou d'individus, et qu'il existait une corrélation entre l'indice de sélection évalué par cette analyse d'image et l'indice de sélection déterminé expérimentalement lors des procédés classiques et longs de sélection décrits ci-avant.

Le procédé de sélection rapide d'espèces, de variétés, de lignées ou d'individus, animaux ou végétaux, par tout caractère génétique selon l'invention consiste à appliquer l'analyse statique d'image de noyaux cellulaires et du faciès chromatinien à l'estimation d'une information génétique, conduisant à l'établissement d'un indice de sélection.

Cette méthode d'analyse, utilisée dans l'application décrite à des frottis cellulaires, et notamment à des frottis du sang des reproducteurs à tester, permet d'estimer l'information génétique contenue dans des paramètres de morphologie, de densitométrie, de texture et de couleur, une analyse multiparamétrique permettant d'apprécier les vecteurs les plus discriminants pour chaque reproducteur.

Les inventeurs ont pu déterminer de façon surprenante qu'il existait une corrélation d'environ 0,8 entre les valeurs génétiques calculées selon les méthodes traditionnelles telles que décrites ci-avant et le procédé selon l'invention.

La présente invention sera mieux comprise et ses avantages ressortiront bien de la description qui suit d'un exemple illustrant le procédé de sélection selon l'invention sans toutefois le limiter.

On utilise un analyseur d'images du type décrit dans AFCET.INTERFACES, N° 50, 5-12, décembre 1986.

Cet appareillage est construit autour d'un organe d'acquisition de l'image, ou capteur ; il dispose d'organes de commande et de traitement distribués dans un processeur interne de commande et de traitement et un processeur externe de traitement et d'analyse des données auquel peuvent être connectés divers périphériques.

Le capteur est constitué d'un microscope équipé d'une platine motorisée et connecté à un dispositif qui digitalise l'image en combinant un procédé de balayage et un photodétecteur.

Le capteur a pour fonction de représenter chaque point d'une image par ses coordonnées et par une (ou plusieurs) valeur(s) numérique(s) correspondant à son niveau de gris dans une (ou plusieurs) longueur-(s) d'onde(s).

Un processeur interne de commande et de traitement permet la réalisation physique des mesures et des calculs qui en dérivent. Constitué par des logiques micro-programmées ou câblées, ce processeur se décompose en deux modules :
- un module de commande du capteur et de gestion de l'acquisition
- un module de traitement et d'analyse des images.

Le module de commande du capteur et de gestion de l'acquisition remplit des fonctions telles que :
- le contrôle des réglages optiques et électroniques du capteur ;
- la focalisation automatique ;
- le déplacement automatique de la préparation ;
- la sélection interactive des échantillons à mesurer ;
- l'acquisition des images numériques ;
- le repositionnement des objets après analyse, etc...

Le module de traitement et d'analyse des images traite les coordonnées et les valeurs numériques des points détectés afin de fournir des paramètres qui décrivent la forme, la densitométrie, la texture et la couleur des objets à analyser.

Un processeur externe de traitement et d'analyse des données (micro-ordinateur) permet de développer les programmes spécifiques à l'application biologique envisagée, de dialoguer avec le système et de réaliser l'exploitation statique des paramètres décrivant les objets rencontrés dans l'échantillon.

Deux méthodes d'analyse peuvent être utilisées en ayant recours soit à un capteur photométrique, soit à un capteur vidéo.

Dans le cas d'un capteur photométrique, le processus de fonctionnement consiste à travailler en mode entièrement automatique selon deux phases successives : une analyse monochromatique à faible résolution pour le repérage des objets à analyser suivie d'une analyse fine mono- ou polychromatique à haute résolution pour leur description quantitative.

L'analyse à faible résolution conduit à la création en mémoire d'un fichier correspondant aux positions X, Y et Z des objets isolés sur la base de paramètres densitométriques et morphologiques.

L'analyse globale d'une zone de la préparation est réalisée par un déplacement en méandres au cours duquel des champs élémentaires adjacents sont balayés et analysés successivement.

L'analyse à haute résolution conduit à la caractérisation paramétrée des objets précédemment repérés et successivement centrés dans le champ du microscope.

Avec le capteur vidéo, l'analyse consiste en un balayage de la lame, champ par champ, au cours duquel les cellules sont analysées.

La séquence globale du traitement et de l'analyse des images est la suivante : segmentation globale de l'image pour isoler les objets à analyser, segmentation interne aux objets pour en distinguer les différents constituants et paramétrisation des objets et de leurs constituants sur la base de traitements appropriés.

La séquence complète d'analyse (comportant l'acquisition, le traitement et l'analyse des images, l'analyse des données et l'édition du résultat) peut être figée et rendue entièrement automatique.

Cette méthode d'analyse d'images permet l'analyse de la texture nucléaire de la chromatine.

Trois types d'informations peuvent être extraites des images nucléaires observées :
- Le degré de compaction des segments de chromatine ou condensation de la chromatine ;
- La proportion relative des différents degrés de compaction de la chromatine, ou distribution de la chromatine ;

- L'arrangement topographique des divers degrés de condensation de la chromatine ou organisation de la chromatine.

La définition structurale des paramètres de texture chromatinienne s'exprime donc en termes de condensation, de distribution et d'organisation de la chromatine.

Trois méthodes de mesure locale de la texture sont plus particulièrement utilisées :

- La caractérisation de l'histogramme des densités optiques du noyau ; six paramètres sont classiquement calculés pour caractériser la distribution de ces densités optiques définissant les propriétés de condensation et de distibution de la chromatine. Ce sont : la surface de l'histogramme, la densité optique intégrée, la densité optique moyenne, l'écart-type, la dissymétrie et l'aplatissement.

- La caractérisation des fonctions de distribution cumulative des densités optiques du noyau, selon une courbe de fonction de distribution cumulative, caractérisée par sept paralètres que l'on peut répartir en trois groupes : les niveaux de gris minimal et maximal qui donnent une estimation sur l'état de condensation de la chromatine, les surfaces partielles qui mesurent respectivement la surface absolue et la surface relative occupée par la chromatine condensée, le seuil de densité optique utilisé étant défini empiriquement comme la valeur médiane des densités optiques pour chaque noyau ; et les pentes qui mesurent le dégré d'homogéneité des niveaux d'intensité soit globalement à l'intérieur du noyau, soit parmi les zones où la chromatine est décondensée, soit parmi les zones où la chromatine est condensée.

- La segmentation intra-nucléaire automatique, obtenue par un algorithme de Fischer selon lequel le critère de minimisation de la somme des variances des classes est utilisé pour partitionner la variable "densité optique" en deux classes ; on détermine ainsi automatiquement un seuil de texture intra-nucléaire situé entre la densité optique maximale et le seuil de segmentation de l'image. On discerne alors trois régions à l'intérieur du noyau, correspondant à trois niveaux différents de condensation de la chromatine : chromatine condensée, chromatine décondensée et chromatine très diffuse.

Ces deux dernières méthodes sont basées sur la description subjective du faciès chromatinien.

Les paramètres de morphologie sont choisis parmi la surface, le périmètre et certains facteurs de forme.

Les paramètres de couleur sont obtenus par une analyse de la teinte, de la luminescence et de la saturation.

Dans l'exemple de mise en oeuvre du procédé selon l'invention, des prélèvements sanguins ont été effectués sur des taureaux en cours de testage, à partir desquels des frottis sur lames ont été réalisés de façon classique, par centrifugation, par exemple par l'appareil HEMASPINNER.

Une moyenne de 10 frottis par animal à tester a été effectuée. Les lames ont été ensuite colorées par la technique MAY GRUNWALD GIEMSA, puis elles ont été observées sous l'analyseur d'images décrit ci-avant qui en effectue l'analyse.

Les images obtenues sont transmises à la carte numérique de traitement d'images, incorporée à l'ordinateur.

L'appareil effectue alors la recherche des cellules (dans le cas de l'exemple, des monocytes) afin d'obtenir, après segmentation et extraction des objets, les informations concernant la morphologie, la densitométrie, la texture et la couleur pour chaque cellule.

Les informations ainsi obtenues sont envoyées dans un fichier paramètre en vue de traitement des données à partir du logiciel de traitement statisque ; ce logiciel établit une régression multiparamètrique par rapport aux données recueillies sur les individus, ce qui permet d'établir un classement desdits individus et de faire la sélection.

L'analyse statistique se fait, de préférence, à partir de 6 paramètres de morphologie et de densitométrie et de 9 paramètres de texture tels que décrits précédemment.

On calcule, pour chaque individu, un indice qui lui est propre.

Le tableau 2 ci-après rassemble les résultats évalués par analyse optique et, comparativement, les résultats calculés à partir des données collectées au cours du testage sur les mêmes individus.

# T A B L E A U  2

| INDIVIDU | INDICE CALCULE | CLASSEMENT | INDICE EVALUE | CLASSEMENT |
|---|---|---|---|---|
| 1 | + 43 | 1 | + 38 | 2 |
| 2 | + 38 | 2 | + 39 | 1 |
| 3 | + 30 | 3 | + 25 | 3 |
| 4 | + 28 | 4 | + 17 | 14 |
| 5 | + 27 | 5 | + 23 | 8 |
| 6 | + 27 | 5 | + 20 | 10 |
| 7 | + 27 | 5 | + 18 | 11 |
| 8 | + 24 | 8 | + 24 | 7 |
| 9 | + 23 | 9 | + 17 | 14 |
| 10 | + 20 | 10 | + 25 | 3 |
| 11 | + 18 | 11 | + 25 | 3 |
| 12 | + 18 | 11 | + 18 | 11 |
| 13 | + 17 | 13 | + 25 | 3 |
| 14 | + 16 | 14 | + 14 | 17 |
| 15 | + 15 | 15 | + 22 | 9 |
| 16 | + 14 | 16 | + 15 | 16 |
| 17 | + 14 | 16 | + 14 | 17 |
| 18 | + 14 | 16 | + 10 | 21 |
| 19 | + 12 | 19 | − 9 | 38 |
| 20 | + 10 | 20 | + 8 | 23 |
| 21 | + 10 | 20 | − 4 | 36 |
| 22 | + 9 | 22 | + 13 | 19 |
| 23 | + 8 | 23 | + 18 | 11 |
| 24 | + 8 | 23 | + 13 | 19 |
| 25 | + 8 | 23 | + 7 | 24 |
| 26 | + 7 | 26 | + 4 | 26 |
| 27 | + 6 | 27 | + 10 | 21 |
| 28 | + 5 | 28 | + 6 | 25 |
| 29 | + 4 | 29 | − 1 | 31 |
| 30 | + 4 | 30 | − 3 | 34 |
| 31 | − 5 | 35 | + 3 | 27 |
| 32 | − 8 | 36 | + 2 | 28 |
| 33 | 0 | 31 | + 2 | 28 |
| 34 | 0 | 31 | − 1 | 31 |

| 35 | − 1 | 33 | − 3 | 34 |
| 36 | − 2 | 34 | + 1 | 30 |
| 37 | − 12 | 37 | − 9 | 38 |
| 38 | − 13 | 38 | − 2 | 33 |
| 39 | − 16 | 39 | − 5 | 37 |
| 40 | − 18 | 40 | − 15 | 40 |
| 41 | − 24 | 41 | − 18 | 41 |

Ce tableau permet de vérifier la bonne corrélation - que l'on peut évaluer à 0,8 entre les indices calculés selon la méthode conventionnelle et les indices évalués par les protocoles faisant l'objet du procédé selon l'invention.

On conçoit tout l'intérêt que présente pour le sélectionneur le procédé selon l'invention ; il permet en effet de raccourcir de façon considérable l'intervalle de génération, les délais actuellement nécessaires à la sélection : ceci est tout spécialement intéressant dans le domaine de la sélection des espèces animales plus spécialement décrites dans ce qui précède.

Ce procédé, mis au point sur des reproducteurs adultes, peut permettre de sélectionner, dès leur jeune âge, les candidats à la reproduction et de limiter les risques pris à la fois par les éleveurs et les organismes de sélection au moment du tri de ces candidats.

Il est bien évident par ailleurs que le procédé selon l'invention ne manque pas de trouver de multiples applications intéressantes lors de la sélection de variétés, de lignées ou d'individus animaux ou végétaux.

## Revendications

1. Procédé se sélection rapide d'espèces, de variétés, de lignées ou d'individus, animaux ou végétaux, pour tout caractère génétique, caractérisé en ce qu'il consiste à appliquer l'analyse statistique d'image de noyaux cellulaire et du faciès chromatinien à l'estimation d'une information génétique.

2. Procédé selon la revendication 1, caractérisé en ce que l'on estime l'information génétique contenue parmi les paramètres de morphologie, de densitométrie, de texture de la chromatine et de couleur.

3. Procédé selon la revendication 2, caractérisé en ce que les paramètres de morphologie sont choisis parmi la surface, le périmètre et certains facteurs de forme.

4. Procédé selon la revendication 2, caractérisé en ce que les paramètres de densitométrie sont choisis parmi la surface de l'histogramme, la densité optique intégrée, la densité optique moyenne, l'écart-type, la dissymétrie et l'aplatissement.

5. Procédé selon la revendciation 2, caractérisé en ce que les paramètres de texture sont choisis parmi les niveaux de gris minimal et maximal, les surfaces partielles, le seuil de densité optique et les pentes.

6. Procédé selon la revendication 2, caractérisé en ce que les paramètres de couleur sont obtenus par une analyse de la teinte, de la luminescence et de la saturation.

7. Procédé selon l'une quelconque des revendications précédentes, caractérisé en ce qu'il comporte une état d'analyse multiparamétrique permettant de déterminer les vecteurs les plus discriminants conduisant à l'établissement d'un indice de sélection.

8. Application du procédé selon l'une quelconque des revendications 1 à 7 à la sélection des espèces animales.

9. Application du procédé selon l'une quelconque des revendications 1 à 7 à la sélection des espèces végétales.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | SU-A-1 450 786 (APPL. MOLECUL. BIOLO.)(15-01-1989) & SOVIET INVENTIONS ILLUSTRATED, semaine 8928, juillet 1989, MOLE, P13, no. 89-205011/28, Derwent Publications Ltd, Londres, GB<br>--- | 1-5,7,9 | G 01 N  15/10<br>G 06 K   9/46<br>A 01 K  67/00<br>A 01 H   1/04 |
| D,Y | CYTOMETRY, vol. 9, 1988, pages 339-348, Alan R. Liss, Inc.; F. GIROUD et al.: "Chromatin texture changes related to proliferation and maturation in erythrocytes"<br>* Page 339, colonne 2, lignes 17-19; page 340, paragraph: "image acquisition"; page 341, colonne 1, lignes 12-18; appendix 2; page 346, colonne 2, lignes 13-26 *<br>--- | 1-5,7-9 | |
| A | APPLIED OPTICS, vol. 26, no. 16, 15 août 1987, pages 3280-3293; J.W. BACUS et al.: "Optical microscope system for standardized cell measurements and analyses"<br>----- | | DOMAINES TECHNIQUES RECHERCHES (Int. Cl.5)<br><br>G 01 N<br>A 01 K<br>A 01 H<br>A 61 D |

Le présent rapport a été établi pour toutes les revendications

| Lieu de la recherche | Date d'achèvement de la recherche | Examinateur |
|---|---|---|
| LA HAYE | 08-10-1990 | HOCQUET A.P.E. |